# EUROPEAN PATENT APPLICATION

(11) **EP 4 741 377 A1**
(43) Date of publication of application: **13.05.2026**
(21) Application number: 24836086.9
(22) Date of filing: 03.07.2024
(51) Int. Cl.: C07D 213/84, C07B 61/00

(54) **METHOD FOR PRODUCING AROMATIC NITRILE COMPOUND AND METHOD FOR PRODUCING CARBONIC ACID ESTER**

(30) Priority: 04.07.2023 JP 2023110314
(71) Applicant: MITSUBISHI GAS CHEMICAL COMPANY, INC., Chiyoda-ku Tokyo 100-8324 (JP)
(72) Inventor: UMEZU Ryotaro, Tokyo 125-8601 (JP); IMADA Shin-ichiro, Tokyo 125-8601 (JP); SHINKAI Yousuke, Tokyo 125-8601 (JP); HARADA Hidefumi, Tokyo 125-8601 (JP); ISOBE Takehiko, Tokyo 125-8601 (JP)
(74) Representative: Hoffmann Eitle
(86) International application number: PCT/JP2024/024147
(87) International publication number: WO 2025/009574

(57) **Abstract**

A method for producing an aromatic nitrile compound is provided by which the aromatic nitrile compound can be selectively obtained in high yield in a shortened reaction time using an aromatic amide compound and which can inhibit the formation of by-products. The above-mentioned problem was solved by the following method for producing an aromatic nitrile compound. The method for producing an aromatic nitrile compound includes a dehydration reaction in which an aromatic amide compound is dehydrated. The dehydration reaction includes a contact step in which the aromatic amide compound and a diluent are brought into contact with a catalyst in a gas phase. In the contact step, the temperature at which the aromatic amide compound and the diluent are brought into contact with the catalyst is 300°C or higher.

## Description

### TECHNICAL FIELD

The present invention relates to a method for producing an aromatic nitrile compound, comprising a step of dehydrating an aromatic amide compound in a gas phase, and to a method for producing a carbonate ester.

### BACKGROUND ART

A carbonate ester is a generic term for a compound in which one or two of the two hydrogen atoms in carbonic acid CO(OH)₂ are replaced by an alkyl or aryl group, and which has the structure RO-C(=O)-OR' (where R and R' each represent a saturated hydrocarbon group or an unsaturated hydrocarbon group).

Carbonate esters are very useful compounds that can be used as additives such as a gasoline additive to improve the octane number, as a diesel fuel additive to reduce particles in exhaust gas, and also as an alkylation agent, a carbonylation agent, a solvent or the like used to synthesize a resin or an organic compound such as a polycarbonate, a urethane, a pharmaceutical, or an agrochemical, or as an electrolyte for lithium ion batteries, a feedstock for a lubricant, or a feedstock for an oxygen absorber to prevent rust on boiler pipes.

Conventionally known methods for producing a carbonate ester, for example, include those involving a step in which water generated as a by-product by the reaction between an alcohol and carbon dioxide is reacted with a nitrile compound (Patent literature 1, 2, etc.). These carbonate ester production methods include a step of dehydrating an amide compound formed from water and the nitrile compound to regenerate the nitrile compound.

Such conventional carbonate ester production methods do not always allow for the selective regeneration of the nitrile compound in a high percent yield in a short period of time, and thus it is not easy to produce the target compound, i.e., a carbonate ester, in an efficient manner.

### PRIOR ART DOCUMENTS

### PATENT DOCUMENTS

Patent literature 1: WO2015/099053
Patent literature 2: WO2020/022416

### SUMMARY OF THE INVENTION

### PROBLEMS TO BE SOLVED BY THE INVENTION

The main objective of the present invention is to provide a method for producing an aromatic nitrile compound using an aromatic amide compound, according to which method an aromatic nitrile compound can be selectively obtained in a high percent yield while shortening the reaction time and also suppressing the generation of by-products. It is also an objective of the present invention to realize a method for effectively producing a carbonate ester by applying such a method for producing an aromatic nitrile compound.

### MEANS FOR SOLVING THE PROBLEMS

The present inventors have examined methods for producing an aromatic nitrile compound which involve dehydrating an aromatic amide compound, and have found that bringing an aromatic amide compound and a diluent into contact with a catalyst in a gas phase at a high temperature significantly improves the reaction rate, shortens the reaction time, and suppresses the generation of by-products, enabling the target compound to be obtained in a high percent yield.

According to the present invention, the regeneration rate of the dehydration reaction that converts an aromatic amide compound into an aromatic nitrile compound can be improved. Furthermore, a method of the present invention for producing a carbonate ester using carbon dioxide and an alcohol, which incorporates such a dehydration reaction step, has been confirmed to have excellent effects, including efficient production of a carbonate ester.

The present invention, for example, includes the following.
[1] A method for producing an aromatic nitrile compound, comprising a dehydration reaction for dehydrating an aromatic amide compound, wherein
   the dehydration reaction comprises a contact step in which the aromatic amide compound and a diluent are brought into contact with a catalyst in a gas phase, and the temperature at which the aromatic amide compound and the diluent are brought into contact with the catalyst in the contact step is 300°C or higher.
[2] The method for producing an aromatic nitrile compound according to [1] above, wherein the diluent comprises at least either a solvent or an inert gas.
[3] The method for producing an aromatic nitrile compound according to [2] above, wherein the solvent comprises at least an aromatic compound.
[4] The method for producing an aromatic nitrile compound according to [3] above, wherein the aromatic compound is represented by the following formula (1): 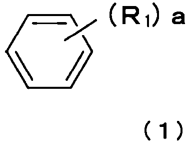 in formula (1),
   R₁ is each independently selected from an optionally substituted C1-C10 alkyl group, an optionally substituted C1-C10 alkoxy group, an optionally substituted C6-C30 aryl group, and an optionally substituted C6-C30 aryloxy group, and
   a is an integer from 1 to 6.
[5] The method for producing an aromatic nitrile compound according to either one of [3] or [4] above, e.g., [3] above, wherein the aromatic compound has one or two aromatic rings and one or more selected from an alkyl group, an alkoxy group, and an aryloxy group.
[6] The method for producing an aromatic nitrile compound according to any one of [3]-[5] above, e.g. [3] above, wherein the aromatic compound has an autoignition temperature of 300°C or higher.
[7] The method for producing an aromatic nitrile compound according to any one of [3]-[6] above, e.g. [3] above, wherein the aromatic compound comprises at least one of 1,3,5-trimethylbenzene, p-cymene, phenyl ether, and anisole.
[8] The method for producing an aromatic nitrile compound according to any one of [1]-[5] above, e.g. [1] above, wherein the pressure of a reaction system in the contact step is 101.3 kPa or less.
[9] The method for producing an aromatic nitrile compound according to any one of [1]-[8] above, e.g., [1] above, wherein the aromatic amide compound comprises at least a heteroaryl amide compound, and the aromatic nitrile compound comprises at least a heteroaryl nitrile compound.
[10] The method for producing an aromatic nitrile compound according to [9] above, wherein the heteroaryl amide compound comprises 2-picolinamide, and the heteroaryl nitrile compound comprises 2-cyanopyridine.
[11] The method for producing an aromatic nitrile compound according to any one of [1]-[10] above, e.g. [1] above, wherein an inert gas in a vaporized state is used in the contact step.
[12] The method for producing an aromatic nitrile compound according to any one of [3]-[11] above, e.g., [11] above, wherein the inert gas comprises at least nitrogen gas.
[13] The method for producing an aromatic nitrile compound according to any one of [1]-[12] above, e.g., [1] above, wherein the catalyst contains an alkali metal.
[14] The method for producing an aromatic nitrile compound according to any one of [2]-[13] above, e.g.[2] above, wherein the solvent is compatible with the aromatic amide compound.
[15] The method for producing an aromatic nitrile compound according to any one of [1]-[14] above, e.g. [1] above, wherein the contact time of the aromatic amide compound with the catalyst in the gas phase is 0.001 seconds or more but less than 10 seconds.
[16] A method for producing a carbonate ester, comprising:
   a first reaction step comprising a carbonate ester-generating reaction in which an alcohol and carbon dioxide are reacted with each other in the presence of an aromatic nitrile compound to generate a carbonate ester and water, and a hydration reaction in which the aromatic nitrile compound is hydrated with the generated water to form an aromatic amide compound; and
   a second reaction step in which the aromatic amide compound is separated from the reaction system of the first reaction step, and then an aromatic nitrile compound is regenerated from the aromatic amide compound by a dehydration reaction for dehydrating the aromatic amide compound, the dehydration reaction involving the contact step according to any one of [1]-[15] above,
   wherein at least a portion of the aromatic nitrile compound regenerated in the second reaction step is used in the first reaction step.
[17] The method for producing a carbonate ester according to [16] above, wherein a catalyst comprising cerium oxide is used in the carbonate ester-generating reaction.
[18] The method for producing a carbonate ester according to either [16] or [17] above, wherein the alcohol comprises a C1-C6 alcohol.

### ADVANTAGEOUS EFFECT OF THE INVENTION

According to the present invention, it is possible to efficiently produce (regenerate) an aromatic nitrile compound, such as cyanopyridine, from an aromatic amide compound, such as a pyridine carboamide (picolinamide, nicotinamide, etc.) or a benzamide. Specifically, the target compound can be selectively obtained in a high percent yield by suppressing the generation of by-products in the dehydration reaction of the aromatic amide compound for the regeneration, thereby improving the reaction rate.

Therefore, according to the present invention, the reaction time of the dehydration reaction for regenerating an aromatic nitrile compound can be significantly shortened compared to conventional methods. Furthermore, the size of the reaction vessel can also be reduced in the dehydration step that involves a gas-phase reaction at a high temperature, compared to the conventional step of dehydrating an amide compound through a liquid-phase reaction or a gas-phase reaction at a lower temperature. According to the present invention, the step of regenerating an aromatic nitrile compound from a corresponding aromatic amide compound can be easily industrialized.

Additionally, according to the present invention, an efficient method for producing a carbonate ester can also be realized by producing an aromatic nitrile compound as described above.

### BRIEF DESCRIPTION OF DRAWINGS

[Figure 1] One example of an apparatus for producing a carbonate ester.
[Figure 2] A schematic diagram of an apparatus for producing an aromatic nitrile compound in an example that uses a solvent.
[Figure 3] A schematic diagram of an apparatus for producing an aromatic nitrile compound in an example that uses an inert gas.
[Figure 4] A diagram showing the relationship between the reaction temperature and the yield of the aromatic nitrile compound (2-cyanopyridine (2-CP)) in the dehydration reaction using a solvent in the examples and comparative examples.
[Figure 5] A diagram showing the relationship between the reaction temperature and the by-product rate of the by-product, pyridine (Py), in the dehydration reaction using a solvent in the examples and comparative examples.

### DESCRIPTION OF EMBODIMENTS

Hereinafter, a suitable embodiment of the present invention is described in detail.

### <1. Method for producing aromatic nitrile compound>

According to a method for producing an aromatic nitrile compound of the present invention, an aromatic amide compound, such as a pyridine carboamide (2-pyridine carboamide, 3-pyridine carboamide, or 4-pyridine carboamide), is dehydrated in a gas phase, thereby converting it into an aromatic nitrile compound, such as cyanopyridine. Specifically, the method for producing an aromatic nitrile compound of the present invention generates an aromatic nitrile compound, for example, by causing a dehydration reaction through a contact step in which an aromatic amide compound is brought into contact with a catalyst supporting a basic metal oxide in a gas phase at a high temperature.

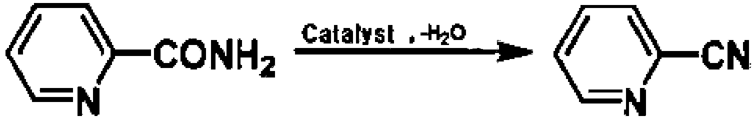

### (Reaction substrate)

Examples of an aromatic amide compound used in the method for producing an aromatic nitrile compound include amide compounds having an aromatic hydrocarbon ring such as a benzene ring, a naphthalene ring, and an anthracene ring, or a heteroaryl ring. Among these aromatic amide compounds, those having a heteroaryl ring, i.e., a heteroaryl amide compound, is suitably used. Examples of the heteroaryl amide compound include amide compounds having a pyridine ring, a pyrimidine ring, a pyridazine ring, a pyrazine ring, a triazine ring, a pyrrole ring, a furan ring, a thiophene ring, an imidazole ring, a pyrazole ring, an oxazole ring, etc.

Specific examples of a preferred heteroaryl amide compound include those having a pyridine ring, such as the pyridine carboamide mentioned above (2-pyridine carboamide, 3-pyridine carboamide, and 4-pyridine carboamide).

As can be appreciated from the above reaction formula, an aromatic nitrile compound produced by the production method of the present invention is a product of a dehydration reaction, corresponding to the aforementioned aromatic amide compound. Therefore, specific examples of the aromatic nitrile compound targeted by the production method of the present invention include a heteroaryl nitrile compound. Examples of the preferred heteroaryl nitrile compound include those having a pyridine ring, such as a cyanopyridine (2-cyanopyridine, 3-cyanopyridine, and 4-cyanopyridine).

### (Catalyst)

The catalyst used in the above dehydration reaction of the present invention preferably contains an oxide of an alkali metal (K, Li, Na, Rb, Cs). In particular, it is preferable to use a catalyst containing an oxide of at least one of Na, K, Rb, and Cs (cesium) as the catalyst used in the above reaction. While a substance generally used as a catalyst support can be used as a support for the above catalyst, examination of various supports shows that it is preferable to contain either SiO₂ or ZrO₂.

The following is an example of a method for producing a catalyst used in the above dehydration reaction of the present invention. If SiO₂ is used as the support, commercially available powdery or spherical SiO₂ can be used. The size of SiO₂ is preferably adjusted, for example, to 4.0 mm or less, to uniformly support the active metal, and pre-calcined in air, for example, at 700°C for about 1 hour, to remove moisture. Although there are SiO₂ with various properties, those with a larger specific surface area are preferable because they allow for greater dispersion of the active metal, thereby increasing the amount of the aromatic nitrile compound generated. Specifically, a specific surface area of 300 m²/g or more is preferred. However, the specific surface area of the catalyst after preparation may be reduced compared to that of SiO₂ alone due to the interaction between SiO₂ and the active metal. In this case, the specific surface area of the catalyst after the production is preferably 150 m²/g or more. A metal oxide as the active species can be supported by an impregnation method such as the incipient wetness method or the evaporation to dryness method.

The metal salt that serves as a catalyst precursor should be water soluble, and various compounds can be used as long as they are alkali metals, such as a carbonate, a hydrogencarbonate, a chloride salt, a nitrate, and a silicate. After impregnation of the support with an aqueous solution of a basic metal precursor, the resultant is dried and calcined to be used as a catalyst. The calcination temperature depends on the precursor used, but it is preferably 400-600°C.

Furthermore, the catalyst used in the present invention is preferably one that has only one or two or more alkali metal oxides supported by a support composed of either one or two of SiO₂ and ZrO₂, but it may contain, along with the above elements, unavoidable impurities that are introduced during the catalyst producing step, etc. However, it is desirable to avoid impurities as much as possible.

The catalyst used in the present invention, in which the metal oxide as the active species is supported by a support, may be in a powder or molded form. In the case of a molded form, it may be in the form of a sphere, pellet, cylinder, ring, wheel, granule, etc. The catalyst may also be fixed to a support structure such as a honeycomb.

While there are no particular restrictions on the size of the support, when a spherical support is used, the average diameter of the catalyst particles (support diameter) is preferably in the range of 0.01-8.0 mm, more preferably 0.03-6.0 mm, and still more preferably 0.05-5.0 mm. Thus, use of a catalyst with a relatively larger support diameter than the average diameter (support diameter) of the catalyst particles used in a liquid-phase reaction ensures the flow path of the reaction substrate in a gas-phase reaction, thereby ensuring a high space velocity. However, a catalyst with a relatively small particle size is preferred when a fluidized bed is used in a gas-phase reaction so that it can be stirred with a small gas flow rate. Conversely, a relatively large particle size or shape is preferred when a fixed bed is used in a gas-phase reaction so that pressure loss is minimized.

The support diameter, i.e., the average diameter of the catalyst particles, refers to the average diameter of the catalyst particles as a whole, including the active ingredient and support of the catalyst. The catalyst support diameter refers to the value measured based on "Test sieving-General requirements" defined in Test sieving: JIS Z8815.

While the amount of the supported catalyst can be determined as desired, the metal-equivalent weight of the active species such as an alkali metal oxide relative to the total catalyst weight is preferably 0.05-2.0 mmol/g, more preferably 0.10-1.5 mmol/g, and still more preferably 0.30-1.0 mmol/g. The amount of catalyst used in the reaction can also be determined as needed.

### (Reaction type and reaction vessel)

According to a method for producing an aromatic nitrile compound of the present invention, a gas-phase reaction in which an amide compound in the form of gas or mist is flown together with a diluent through a catalyst bed is preferably adopted, where, for example, a fixed-bed or fluidized-bed gas-phase reactor can be suitably used. Thus, in the contact step in which the aromatic amide compound is brought into contact with the catalyst in the gas phase, it is preferable to use an aromatic amide compound in a completely vaporized state, but it is also acceptable to use an aromatic amide compound in a state where droplets (mist) are partially contained in the gas.

It is desirable to carry out the method for producing an aromatic nitrile compound while removing the generated by-product water through a dehydration reaction. The present inventors have diligently studied and found that the amount of the aromatic nitrile compound can be improved while suppressing the generation of the by-products, for example, by attaching a vaporization chamber above the reaction tube containing the catalyst, dropping an aromatic amide compound into the vaporization chamber, and allowing the amide compound to pass through the catalyst bed in the gas phase within a short period of time.

### (Diluent)

In the above dehydration reaction, it is preferable to use a diluent. Specifically, at least either an inert gas or a solvent described in detail below, is preferably used as the diluent in the dehydration reaction. The diluent is mainly used to reduce generation of by-products such as pyridine by reducing the frequency of contact between water, which is generated when the aromatic amide compound is vaporized and reacted with the catalyst, and an unreacted aromatic amide compound and an aromatic nitrile compound, i.e., the reaction product.

Therefore, an inert gas can also be used as a diluent. If the diluent is compatible with the aromatic amide compound in a liquid phase, it can also prevent blockage trouble caused by precipitation of the aromatic amide compound, by mixing the aromatic amide compound with the diluent that is compatible with the aromatic amide compound in advance and feeding the solution in the step of vaporizing the aromatic amide compound. Specifically, an inert gas may be used as the diluent, or a liquid that is incompatible with the aromatic amide compound targeted by the dehydration reaction may be used as the diluent. More preferably, a compatible liquid is used. A diluent that is compatible with an aromatic amide compound in a liquid phase is not only a diluent that can be mixed with the aromatic amide compound at any ratio, but also a diluent that can dissolve the target aromatic amide compound only up to a predetermined solubility, and that can dissolve the aromatic amide compound to a concentration equal to or below the upper limit of the said solubility at a predetermined temperature.

### (Inert gas)

As described above, in the dehydration reaction, an inert gas, along with the aromatic amide compound, is preferably brought into contact with the catalyst in a gas phase. Specifically, the inert gas is used in a vaporized state in the dehydration reaction. Specific examples of the inert gas used in the dehydration reaction include nitrogen gas and a rare gas such as helium and argon, preferably nitrogen gas. The flow rate of the inert gas and the like are described below.

### (Solvent)

In the above dehydration reaction, a solvent is preferably used. In particular, as described above, the solvent can prevent blockage trouble caused by precipitation of the aromatic amide compound, by mixing the aromatic amide compound with the solvent that is compatible with the aromatic amide compound in advance and feeding the solution in the step of vaporizing the aromatic amide compound. Specifically, the solvent is preferably one that is compatible with the aromatic amide compound targeted by the dehydration reaction. A solvent that is compatible with an aromatic amide compound is not only a solvent that can be mixed with the aromatic amide compound at any ratio, but also a solvent that can dissolve the target aromatic amide compound only up to a predetermined solubility, and that can dissolve the aromatic amide compound to a concentration equal to or below the upper limit of the said solubility at a predetermined temperature.

The solvent preferably contains at least an aromatic compound, which is preferably one represented by the following formula (1).

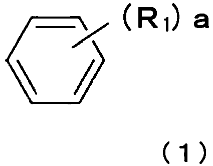

In formula (1), R₁ are each independently selected from an optionally substituted C1-C10 alkyl group, an optionally substituted C1-C10 alkoxy group, an optionally substituted C6-C30 aryl group, and an optionally substituted C6-C30 aryloxy group.

R₁ in formula (1) is preferably an optionally substituted C1-C6 alkyl group, an optionally substituted C1-C6 alkoxy group, or an optionally substituted C6-C18 aryl group. R₁ is more preferably any of an optionally substituted C1-C4 alkyl group, an optionally substituted C1-C3 alkoxy group, and an optionally substituted C6-C12 aryl group, and still more preferably any of a C1-C3 alkyl group, an optionally substituted C1 or C2 alkoxy group, and an optionally substituted C6-C8 aryl group.

In formula (1), a is an integer from 1 to 6, where a is preferably an integer from 1 to 4 and more preferably an integer from 1 to 3.

Examples of the substituent mentioned above include a halogen atom, a hydroxy group, a carboxy group, a cyano group, a C1-C10 amide group, a C1-C10 alkyl group, a C5-C10 cycloalkyl group, and a C1-C10 alkoxy group. The number of carbon atoms relating to general formula (1) refers to the total number of carbon atoms, including the number of carbon atoms in the substituent.

The aromatic compound as the solvent preferably has one or two aromatic rings and one or more selected from an alkyl group, an alkoxy group, and an aryloxy group, and more preferably a total of 1-3 alkyl and/or alkoxy groups. The aromatic compound also preferably has multiple (e.g., two) aromatic rings, such as benzene rings, connected by an ether bond.

The aromatic compound of the solvent used in the dehydration reaction preferably contains, for example, at least one of 1,3,5-trimethylbenzene, p-cymene, phenyl ether, and anisole. Other specific examples of the solvent include an aromatic compound including a pyridine compound, a ketone compound, an ether compound, an ester compound, and an alcohol. Preferably, an aromatic compounds mentioned above is used.

Examples of the pyridine compound, i.e., a compound with a pyridine backbone, as the solvent include a 2-alkylpyridine, a 3-alkylpyridine, and a 4-alkylpyridine, such as 4-methylpyridine and pyridine. In particular, when carrying out a dehydration reaction that may generate pyridine as a by-product, using a pyridine compound as a solvent under suitable conditions set in advance can eliminate the need for a step of removing pyridine, i.e., the main by-product.

Examples of the ketone compound as the solvent include a cyclic ketone compound such as cyclopentanone and cyclohexane, and acetone.

It is preferable to use a solvent consisting of only one or more substances selected from the above-mentioned compounds as the solvent in the dehydration reaction, but a mixed solvent further containing other compounds may also be used.

The solvent is preferably vaporized together with the aromatic amide compound and used in the dehydration reaction (contact step). Thus, it is preferable to employ a solvent that can be vaporized and used in the dehydration reaction. In addition, the solvent, which is preferably an aromatic compound, preferably have a high autoignition temperature because it is used in a dehydration reaction that takes place at a high temperature, as described in detail below. For example, the solvent preferably has an autoignition temperature of 300°C or higher, 340°C or higher, 380°C or higher, or 400°C or higher, more preferably 430°C or higher or 450°C or higher, still more preferably 470°C or higher or 500°C or higher, and particularly preferably 550°C or higher.

While the boiling point of the solvent, which is preferably an aromatic compound, is not particularly limited, the boiling point at an ambient pressure is, for example, 20°C or higher but 300°C or lower, preferably 80°C or higher but 250°C or lower, and still more preferably 110°C or higher but 200°C or lower.

### (Conditions for dehydration reaction including contact step)

The method for producing an aromatic nitrile compound, as described above, involves a contact step in which an aromatic amide compound is brought into contact with a catalyst in a gas phase in a dehydration reaction.

In the contact step, the temperature at which the aromatic amide compound and the diluent such as a solvent are brought into contact with the catalyst is, for example, 285°C or higher, preferably 300°C or higher. The contact temperature is more preferably 300°C or higher but 550°C or lower, still more preferably 320°C or higher but 520°C or lower, and particularly preferably 350°C or higher but 490°C or lower.

The temperature at which the aromatic amide compound and the like are brought into contact with the catalyst is, for example, the temperature in the vicinity of the catalyst bed to which the catalyst is fixed in the reaction tube (reaction vessel), as described below.

The contact time of the aromatic amide compound with the catalyst in the contact step, or, when an inert gas, a solvent, etc. is contained along with the aromatic amide compound, the contact time of the mixed gas of this gaseous composition, is preferably 0.001 seconds or more, or 0.005 seconds or more, for example, 0.01 seconds or more but less than 10 seconds. The above contact time with the catalyst is more preferably 0.1 seconds or more but less than 5 seconds, and still more preferably 0.5 seconds or more but less than 2 seconds.

The contact time with the catalyst refers to the average time of the aromatic amide compound gas or the above mixed gas to pass through the catalyst bed, and is calculated by the following equation: Contact time with catalyst (sec) = Height of catalyst bed in reactor (cm)/Gas linear velocity in reactor (cm/sec).

In the contact step, the flow rate of the aromatic amide compound to the flow rate of the gaseous composition in mole ratio is preferably 1:0-1:200, more preferably 1:1-1:100, and still more preferably 1:1-1:20.

The space velocity (SV) of the total gas component in the contact step is preferably 1,000-50,000 (h⁻¹), more preferably 1,500-30,000 (h⁻¹), and still more preferably 2,000-25,000 (h⁻¹). When the contact step is carried out under a reduced pressure, the space velocity (SV) of the total gas component is preferably 1,000-1,000,000 (h⁻¹), more preferably 1,500-700,000 (h⁻¹), and still more preferably 1,900-504,000 (h⁻¹).

The dehydration reaction can be performed under the conditions of a pressurized (e.g., 506.5 (kPa)) to ambient (101.3 (kPa)) or reduced (e.g., 101.3-1.0 (kPa) or less, 101.3-1.0 (kPa)-0.1 (kPa), etc.) pressure. Although the conditions are not limited to these, it is preferable to perform the dehydration reaction under a reduced pressure.

The reaction pressure in the dehydration reaction is preferably 101.3 (kPa) or less, more preferably 90 (kPa)-1.0 (kPa) or 80 (kPa)-5.0 (kPa), still more preferably 70 (kPa)-10 (kPa) or 60 (kPa)-20 (kPa), and particularly preferably 50 (kPa)-30 (kPa).

In addition, the aromatic amide compound is preferably dehydrated in the liquid state before vaporization. When using a molecular sieve as a dehydrating agent, there are no particular restrictions on its type or shape. For example, spherical or pellet-shaped sieves of 3A, 4A, 5A, etc., which are generally highly water absorbent, can be used. For example, Zeolum from Tosoh Corporation can be used favorably.

### (Example of by-product in dehydration reaction)

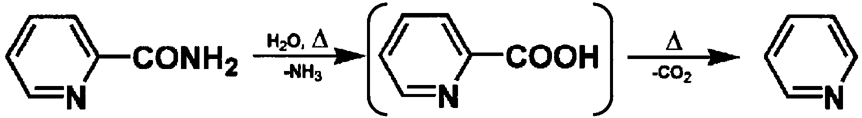

In the dehydration reaction of an aromatic amide compound, pyridine may be produced as a by-product of the aforementioned decomposition of the aromatic amide compound via an aromatic carboxylic acid. However, the reaction solution after the dehydration reaction using the contact step of the present invention contains almost no by-product, such as pyridine, as shown in the above formula.

### <2. Method for producing carbonate ester using aromatic nitrile compound>

When an aromatic nitrile compound is regenerated from an aromatic amide compound through a dehydration reaction, the reaction rate is significantly improved and the reaction time is significantly shortened by adding a contact step in a gas phase. This made it possible to balance the regeneration rate of an aromatic nitrile compound from an aromatic amide compound through a dehydration reaction with the synthesis rate of a carbonate ester from CO₂ and an alcohol using the aromatic nitrile compound, thereby establishing these reactions as a series of commercial processes. Accordingly, the present inventors were able to apply this finding to the method for producing a carbonate ester and conceived of a method for producing a carbonate ester, which is described below.

### (First reaction step)

A first reaction step in the method for producing a carbonate ester of the present invention includes a reaction (carbonate ester-generating reaction) in which a carbonate ester is produced by directly reacting an alcohol with carbon dioxide in the presence of a solid catalyst containing CeO₂ (cerium oxide) or the like and an aromatic nitrile compound.

In this step, when a reaction takes place between an alcohol and carbon dioxide, water is generated along with a carbonate ester. However, an aromatic amide compound is generated by the hydration reaction between the aromatic nitrile compound present in the reaction system and the generated water, thereby removing or reducing the generated water from the reaction system. Thus, generation of a carbonate ester can be accelerated by efficiently removing water from the reaction system. For example, it can be represented by the following formula.

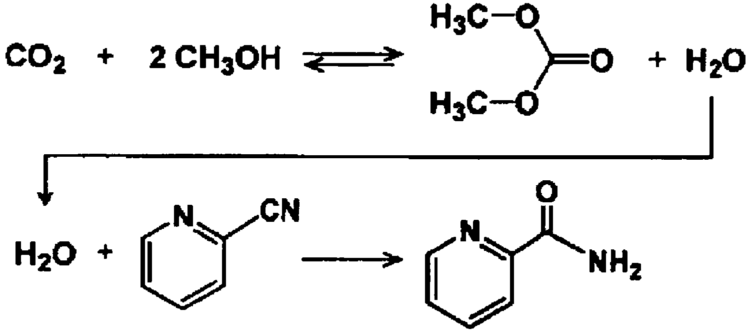

### (Alcohol)

As the alcohol, any alcohol selected from one or two or more of a primary alcohol, a secondary alcohol, and a tertiary alcohol can be used. For example, methanol, ethanol, 1-propanol, isopropanol, 1-butanol, 1-pentanol, 1-hexanol, 1-heptanol, 1-octanol, 1-nonanol, allyl alcohol, 2-methyl-1-propanol, cyclohexanemethanol, benzyl alcohol, ethylene glycol, 1,2-propanediol, and 1,3-propanediol are preferred because they result in high product percent yields and high reaction rates. In these cases, the generated carbonate esters are dimethyl carbonate, diethyl carbonate, dipropyl carbonate, diisopropyl carbonate, dibutyl carbonate, dipentyl carbonate, dihexyl carbonate, diheptyl carbonate, dioctyl carbonate, dinonane carbonate, diallyl carbonate, di-2-methyl-propyl carbonate, dicyclohexanemethyl carbonate, dibenzyl carbonate, ethylene carbonate, 1,2-propylene carbonate, and 1,3-propylene carbonate, respectively.

In the first reaction step, it is preferable to use a C1-C6 alcohol, and it is more preferable to use a C2-C4 alcohol. Specifically, when using the resulting carbonate ester as a feedstock for diaryl carbonate, it is preferable to adjust the number of carbon atoms in the alcohol to fall within the aforementioned range.

Moreover, in the first reaction step, it is preferable to use a monohydric or dihydric alcohol.

### (Carbonate ester-producing catalyst)

In the first reaction step for producing an carbonate ester, it is preferable to use a solid catalyst of either or both CeO₂ and ZrO₂. For example, it is preferable to use CeO₂ alone, ZrO₂ alone, a mixture of CeO₂ and ZrO₂, or a solid solution or composite oxide of CeO₂ and ZrO₂, and it is particularly preferable to use CeO₂ alone. For a solid solution or composite oxide of CeO₂ and ZrO₂, the mixing ratio of CeO₂ and ZrO₂ is typically 50:50, but can be determined as desired.

The catalyst used in the first reaction step may be in either a powder or molded form. In the case of a molded form, it may be in the form of a sphere, pellet, cylinder, ring, wheel, granule, etc.

### (Carbon dioxide)

The carbon dioxide that can be used in the present invention may not only be one prepared as an industrial gas, but may also be one separated and recovered from the exhaust gas emitted by a factory manufacturing products, a steel mill, a power plant, or the like.

### (Solvent in carbonate ester-generating reaction)

In the carbonate ester-generating reaction, it is preferable to use a solvent with a higher boiling point than that of the amide compound being generated. More preferably, the solvent used in the carbonate ester-generating reaction contains at least one of dialkylbenzene, alkylnaphthalene, or diphenylbenzene. Specific examples include Barrel Process Oil B28AN and Barrel Process Oil B30 (from Matsumura Oil Co., Ltd.), which contain a component such as dialkylbenzene, alkylnaphthalene, or diphenylbenzene.

### (Distillation separation)

After the reaction, the products can be recovered through distillation separation of the main product, i.e., the carbonate ester, the by-product, i.e., the aromatic amide compound, the unreacted aromatic nitrile compound, and the solid catalyst such as CeO₂.

### (Second reaction step)

Next, a second reaction step of the present invention is described. In the second reaction step, the aromatic amide compound produced as a by-product in the first reaction step is separated from the system, preferably after the carbonate ester-generating reaction, and then subjected to a dehydration reaction to produce an aromatic nitrile compound. The second reaction step corresponds to the method for producing an aromatic nitrile compound described above. Specifically, in the second reaction step for generating a carbonate ester, an aromatic nitrile compound is produced from an aromatic amide compound using the process described in the above section on the method for producing an aromatic nitrile compound, thereby regeneraing an aromatic nitrile compound. Accordingly, the details of the second reaction step are omitted.

### (Reuse of aromatic nitrile compound)

The aromatic nitrile compound regenerated in the second reaction step can be reused in the first reaction step (hydration reaction).

As described above, according to the present invention, it is possible to efficiently regenerate an aromatic nitrile compound from an aromatic amide compound while suppressing the generation of by-products by conducting the reaction in a gas phase in the dehydration reaction of the aromatic amide compound. Furthermore, for example, fixing the catalyst inside the reaction tube eliminates the need for a solid-liquid separation step of the catalyst and makes the recovery of the aromatic nitrile compound easier. Thus, in the present invention, it is possible to selectively regenerate an aromatic nitrile compound from the aromatic amide compound and to proceed a series of reactions while separating each component by distillation separation only, without the need for solid-liquid separation of the catalyst, thereby realizing an efficient process, as described in detail below.

### <3. Apparatus for producing carbonate ester>

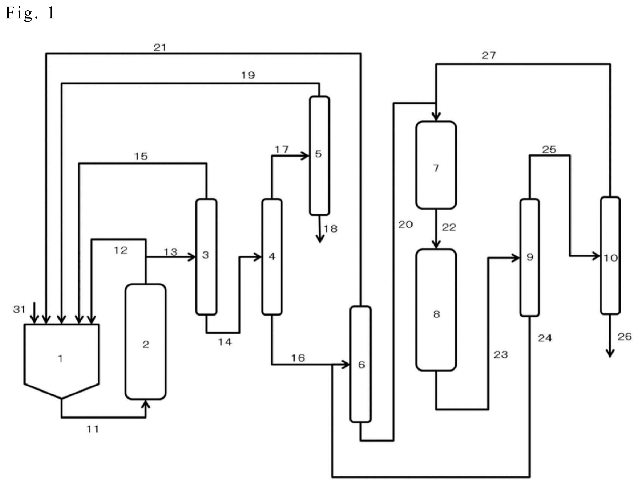

Next, an apparatus for producing a carbonate ester used in the present invention is described in more detail by way of specific examples shown below. Figure 1 is one example of suitable equipment.

### (First reaction step)

In the first reaction step, feedstocks, namely, an alcohol (1-propanol (PrOH); liquid phase), 2-cyanopyridine (2-CP; liquid phase), and carbon dioxide (CO₂; liquid phase, which may be fed via a boost pump), are continuously fed to a buffer tank 1 via a feed pipe 31, a CO₂ transfer pipe 15 at the top of a CO₂ recovery tower (CO₂ transfer pipe 15 on the top side of the CO₂ recovery tower), a liquid transfer pipe 19 at the top of a carbonate ester recovery tower (liquid transfer pipe 19 on the top side of the carbonate ester recovery tower), and a liquid transfer pipe 21 at the top of an amide separation tower (liquid transfer pipe 21 on the top side of the amide separation tower). This feedstock mixture is circulated between a flow-type fixed-bed carbonate ester reactor 2 (first reaction unit), which has a solid catalyst (solid phase) (e.g., either or both CeO₂ and ZrO₂) fixed to a support material, and the buffer tank 1, via first and second reaction solution circulation pipes 11 and 12 using a pump (not shown), thereby synthesizing dipropyl carbonate (DPrC). The reaction solution containing DPrC is continuously withdrawn from the second reaction solution circulating pipe 12 for the same amount as the amount of the feedstocks fed to the buffer tank 1, recovered via a pipe 13, and sent to a DPrC recovery step. PrOH and CO₂ can be recovered from the reaction solution and reused by transferring them to the buffer tank 1 via the liquid transfer pipe 19 at the top of the carbonate ester recovery tower and the CO₂ transfer pipe 15 at the top of the CO₂ recovery tower, respectively. Although a new material is used as 2-cyanopyridine, for example, at the start of the reaction, 2-cyanopyridine regenerated from 2-picolinamide may be reused by separating and purifying it in an amide separation tower 6 and transferring it to the buffer tank 1 via the liquid transfer pipe 21 at the top of the amide separation tower.

As a direct synthesizer of a carbonate ester (carbonate ester reactor 2) that uses, for example, CeO₂, ZrO₂, etc. as a solid catalyst, any of a batch reactor, a semi-batch reactor, or a flow reactor such as a continuous tank reactor or a tubular reactor may be used, but a fixed-bed reactor is preferred because the catalyst does not need to be filtrated/separated if it is fixed inside the reactor.

### (Temperature of reaction solution)

The temperature of the reaction solution in the carbonate ester reactor 2 is preferably 50-300°C. If the temperature of the reaction solution is below 50°C, the reaction rate is low, and both the carbonate ester synthesis reaction and the hydration reaction with 2-cyanopyridine may be difficult to proceed, resulting in a tendency of low carbonate ester productivity. If the temperature of the reaction solution exceeds 300°C, the reaction rate of each reaction increases, but the carbonate ester decomposes or denatures easily, and the aromatic amide compound, such as 2-picolynamide, reacts easily with the alcohol, resulting in a tendency of a low carbonate ester percent yield. The temperature of the reaction solution is more preferably 100-150°C. However, since the ideal temperature of the reaction solution is considered to vary depending on the type and amount of the solid catalyst and the amount and ratio of the feedstocks (alcohol, 2-cyanopyridine), the optimal conditions are preferably determined accordingly. Since the preferred temperature of the reaction solution is 100-150°C, it is desirable to preheat the feedstocks (alcohol, 2-cyanopyridine, etc.) with steam, etc. at an earlier stage in the carbonate ester reactor.

### (Reaction pressure)

The reaction pressure in the carbonate ester reactor 2 is preferably 0.1-20 MPa (absolute pressure). If the reaction pressure is lower than 0.1 MPa (absolute pressure), a decompression device will be required, which not only complicates the equipment and increases the cost, but also requires power energy for decreasing the pressure, thereby rendering the energy efficiency poor. Conversely, if the reaction pressure exceeds 20 MPa, the hydration reaction with 2-cyanopyridine may be difficult to proceed, which not only lowers the percent yield of the carbonate ester, but also requires power energy for increasing the pressure, thereby deteriorating the energy efficiency. From the viewpoint of increasing the percent yield of the carbonate ester, the reaction pressure is more preferably 0.5-15 Mpa (absolute pressure), and still more preferably 0.1-10 MPa (absolute pressure).

### (Used amount of aromatic nitrile compound (e.g. 2-cyanopyridine, etc.))

For the aromatic nitrile compound such as 2-cyanopyridine used in the hydration reaction, it is preferable to use it in a molar amount that is 0.1 times or more but 5 times or less the theoretical molar amount of water produced as a by-product in the reaction between the alcohol and CO₂ as feedstocks, and it is desirable to introduce the compound into the reactor in advance before the reaction. More desirably, the molar amount of the aromatic nitrile compound such as 2-cyanopyridine is 0.2 times or more but 3 times or less the theoretical molar amount of water produced as a by-product in the reaction between the alcohol and CO₂ as feedstocks, and particularly desirably 0.3 times or more but 1.5 times or less. If the molar amount of the aromatic nitrile compound such as 2-cyanopyridine is too small, the percent yield of the carbonate ester may be poor because there is less 2-cyanopyridine or the like contributing to the hydration reaction. On the other hand, if the aromatic nitrile compound such as 2-cyanopyridine is introduced in an excess molar amount compared to that of the alcohol used as a feedstock, side reactions of 2-cyanopyridine or the like increase, which is undesirable. Furthermore, since the ideal amounts of the alcohol, 2-cyanopyridine etc., relative to the solid catalyst are considered to vary depending on the type and amount of the solid catalyst, the type of the alcohol, and the ratio to 2-cyanopyridine, the optimal conditions are preferably determined accordingly.

### (Separation of reaction product)

Separation of the reaction product is preferably all done by distillation. After the reaction in the carbonate ester reactor 2, a reaction solution 13 is sent to a CO₂ recovery tower 3, and a mixture of PrOH, DPrC, 2-cyanopyridine, and 2-picolinamide is recovered from the bottom of the CO₂ recovery tower 3 via a liquid transfer pipe 14 at the bottom of the CO₂ recovery tower, while CO₂ is recovered from the top of the CO₂ recovery tower 3 via the CO₂ transfer pipe 15 at the top of the CO₂ recovery tower. The recovered CO₂ is sent to the buffer tank 1 and recycled for a reaction in the carbonate ester reactor 2.

The mixture recovered from the CO₂ recovery tower 3 is sent to a dehydrating agent separation tower 4 via the liquid transfer pipe 14 at the bottom of the CO₂ recovery tower. A mixture of 2-cyanopyridine and 2-picolinamide is recovered from the bottom of the dehydrating agent separation tower 4 via a liquid transfer pipe 16 at the bottom of the dehydrating agent separation tower, and PrOH and DPrC are recovered from the top of the dehydrating agent separation tower 4 via a liquid transfer pipe 17 at the top of the dehydrating agent separation tower.

The mixture recovered from the bottom of the dehydrating agent tower 4 is sent to the amide separation tower 6 via the liquid transfer pipe 16 at the bottom of the dehydrating agent separation tower, and 2-picolinamide is recovered from the bottom of the amide separation tower 6 (20) while 2-cyanopyridine is recovered from the top of the amide separation tower 6. The recovered 2-cyanopyridine is sent to the buffer tank 1 via the liquid transfer pipe 21 at the top of the amide separation tower, and recycled for a reaction in the carbonate ester reactor 2.

PrOH and DPrC recovered from the top of the dehydrating agent separation tower 4 are sent to a carbonate ester recovery tower 5 via the liquid transfer pipe 17 at the top of the dehydrating agent separation tower. DprC is recovered from the bottom of the carbonate ester recovery tower 5 via a liquid transfer pipe 18 at the bottom of the carbonate ester recovery tower while PrOH is recovered from the top of the carbonate ester recovery tower 5 via a liquid transfer pipe 19 at the top of the carbonate ester recovery tower. The recovered PrOH is sent to the buffer tank 1 and recycled for a reaction in the carbonate ester reactor 2.

### (Second reaction step)

In the second reaction step, 2-cyanopyridine is generated by a dehydration reaction of 2-picolinamide in a nitrile regeneration gas-phase reactor 8.

2-Picolinamide recovered in the amide separation tower 6 is transferred to a vaporizer 7 via a liquid transfer pipe 20 at the bottom of the amide separation tower, preferably mixed with an inert gas such as nitrogen, and further heated to near the boiling point of the amide compound to make a gas or a gas-droplet mixture, which is then transferred to the nitrile regeneration gas-phase reactor 8 via a vaporizer-nitrile regeneration gas-phase reactor connecting pipe 22. The type of vaporizer 7 used for vaporizing the amide compound, etc. is not particularly limited, and any vaporizer, such as an ejector type vaporizer, a contact type vaporizer, or a bubbling device, may be used.

In the apparatus for producing a nitrile compound (nitrile regeneration gas-phase reactor 8) used in the present invention, 2-picolinamide, preferably along with an inert gas such as nitrogen, is brought into contact with a catalyst supporting a basic metal oxide in the gas phase to cause a dehydration reaction of 2-picolinamide. This dehydration reaction produces 2-cyanopyridine. When transferring the amide compound, it can be dissolved in an amide compound transferring solvent to prevent blockage trouble. When the amide transferring solvent is used, the solvent is preferably vaporized together with the amide to carry out the nitrile regeneration gas-phase reaction, in which case solvent vapor can be used instead of an inert gas.

While the type of the nitrile regeneration gas-phase reactor 8 is not particularly limited, a gas-phase reaction in which an amide compound in the form of gas or mist is flown together with an inert gas, etc. through a catalyst bed is preferable, where a gas-phase reactor with a fixed-bed, a fluidized-bed, or the like can be suitably used.

In order to efficiently proceed the dehydration reaction by bringing 2-picolinamide into contact with the catalyst, etc. in the gas phase, the various reaction conditions described above for the dehydration reaction or the reaction conditions described in EXAMPLES below are employed as appropriate.

Mixed gas containing 2-cyanopyridine is transferred from the gas-phase reactor 8 to a H₂O separator 9 via a gas-phase reaction product transfer pipe 23. Water and nitrogen gas are separated from the mixed gas in the H₂O separator 9, and the recovered 2-cyanopyridine and 2-picolineamide are transferred from the H₂O separator 9 to the amide separation tower 6 via a H₂O separator high boiling transfer pipe 24. 2-Picolinamide is recovered from the bottom of the amide separation tower 6 via a liquid transfer pipe 20 at the bottom of the amide separation tower and transferred to the vaporizer 7 while 2-cyanopyridine is recovered from the top of the amide separation tower 6. The recovered 2-cyanopyridine is sent to the buffer tank 1 via the liquid transfer pipe 21 at the top of the amide separation tower, and recycled for a reaction in the carbonate ester reactor 2.

Furthermore, nitrogen gas and water separated in the H₂O separator 9 are sent to a N₂ recovery device 10 via a H₂O separator lower boiling transfer pipe 25 to separate water and recover nitrogen gas in the N₂ recovery device 10. Water separated in the N₂ recovery device 10 is sent outside the carbonate ester apparatus via a N₂ recovery device moisture transfer pipe 26. Nitrogen gas recovered in the N₂ recovery device 10 can be transferred to the vaporizer 7 via a N₂ recovery device N₂ transfer pipe 27 to be used for a gas-phase reaction. If a solvent is used for transferring an amide compound, a separate step for recovering the solvent can be provided so that it can be reused for transferring an amide compound. The types of the H₂O separator 9 and the N₂ recovery device 10 are not particularly limited, and any device, such as a cooling device or a membrane separator, may be used.

As described above, according to the present invention, not only the dehydration of an amide compound can proceed in the contact step in a gas phase, but it is also possible to separate the reaction product and the compound to be reused by distillation separation or the like, without requiring solid-liquid separation. Therefore, according to the present invention, a carbonate ester can be efficiently produced with fewer production steps while simplifying the equipment.

### EXAMPLES

Hereinafter, the present invention is described in more detail by way of examples, although the present invention is not particularly limited to these examples. First, examples and comparative examples of a method for producing cyanopyridine are described.

### Example 1 (Example using solvent as diluent)

SiO₂ (from Fuji Silicia Chemical, CARiACT, G-6 (support diameter 0.010 mm)) as a support was pre-calcined at 700°C for 1 hour. Subsequently, to support Cs as an alkali metal, an aqueous solution was prepared using CsNO₃ (from Wako Pure Chemical Industries, Ltd.) so that the final amount of the supported Cs metal was 0.5 mmol/g, and the aqueous solution was used to impregnate SiO₂. The resultant was then dried at 110°C for 6 hours and calcined at 500°C for 3 hours to obtain a Cs₂O/SiO₂ catalyst.

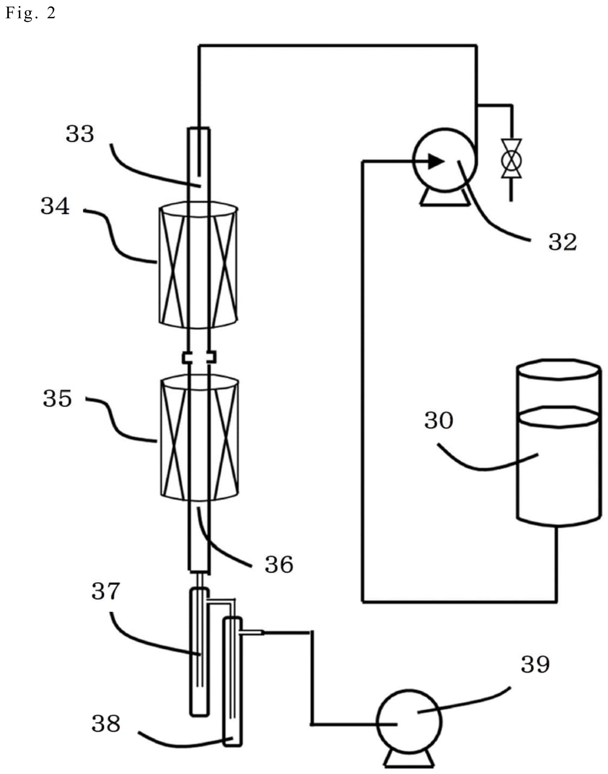

The catalyst produced by the method described above was packed into a reaction tube 36 made of SUS316 with an inner diameter of 10.8 mm and a length of 35 cm (see Figure 2). Furthermore, a vaporization chamber 33 made of SUS316 with an inner diameter of 10.8 mm and a length of 35 cm, packed with Raschig rings, was placed directly above the reaction tube 36.

203 g of 2-picolinamide (2-PA) was heated and mixed with 1,3,5-trimethylbenzene (200 g) as a transfer solvent in an oil bath at 140°C. The resulting mixed solution of 2-PA and 1,3,5-trimethylbenzene was transferred to a feedstock vessel 30.

The vaporization chamber 33, connected to the feedstock vessel 30, and the reaction tube 36 were heated in tube furnaces 34 and 35 (see Figure 2). The outlet pipe of the reaction tube 36 was kept warm at 180°C. A dry ice/methanol cooling trap vessel 37 and a trap vessel 38 were installed to collect the reactants. The outlet pipe of the trap vessel 38 was connected to a vacuum pump 39, and the pressure in the vaporization chamber 33 and the reaction tube 36 was reduced to 40 (KPa).

After the temperature of the vaporization chamber and the reaction tube was stabilized at 337°C, a plunger pump 32 was initiated to carry out the reaction for the time indicated in the column "Catalyst contact time" in Table 1 below.

### Examples 2-10 and Comparative examples 1-7

Using the same reactor as in Example 1, a dehydration reaction of 2-picolinamide was carried out in the same manner as in Example 1, except that the reaction conditions were changed as shown in Table 1 below.

The conditions for the above contact reaction (dehydration reaction) are shown in Table 1. After the reaction was completed, the reactants were collected and analyzed by GC-FID.

The analytical conditions for the results of the contact reaction (dehydration reaction) were as follows.

### [Analytical conditions]

### (GC-FID)

Shimadzu GC-2014, column: TC-17 (length 30 m, inner diameter 0.25 mm ID, thickness of liquid phase 0.25 µm),
Temperature of vaporization chamber: 250°C, detector: 250°C, support N₂: 175 kPa, column flow rate: 10.3 mL/min, split ratio: 5.0
Temperature program: [hold at 40°C for 10 min] → [increase to 250°C, 12°C/min] → [hold at 250°C for 5 min]

### [24-Hour yield per gram of catalyst (mmol/24 hr·g)] =

(Amount of product recovered after 24 hours of reaction (mmol))/(Amount of catalyst (g))
[Space velocity: SV (hr⁻¹)] = (Amount of gas passing through catalyst bed (L·hr⁻¹))/(Amount of catalyst (L))
Amount of gas: Sum of gas volumes of nitrogen, 2-PA, and solvent (L·hr⁻¹)
[Height of packed catalyst (height of catalyst bed in reactor)]: Height of catalyst packed in reaction tube (cm)
[Catalyst contact time (sec)] = [Height of packed catalyst (cm)]/[Linear velocity (gas linear velocity in reactor) (cm/sec)]

### [Catalyst support diameter]

The catalyst support diameter refers to the value measured based on "Test sieving-General requirements" defined in Test sieving: JIS Z8815.

### [Autoignition temperature of solvent]

The autoignition temperatures of the solvents used in the examples and comparative examples were measured values or literature values. The measured values of autoignition temperature were determined by an ignition point test in accordance with ASTM E659-15.

### [Vaporization chamber/reaction tube temperature and reaction temperature (contact temperature)]

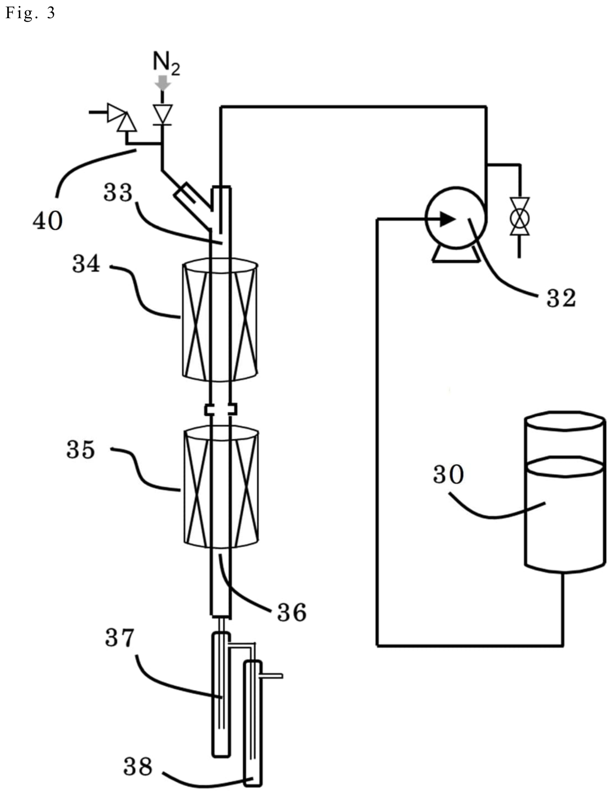

The temperature of the vaporization chamber and reaction tube and the reaction temperature in the dehydration reaction were measured by inserting a sheath tube made of SUS316 with a built-in thermometer from the bottom of the reaction tube, which is shown, for example, as the reaction tube 36 in the schematic diagram of the reactor in Figures 2 and 3, to a position just below the catalyst bed. Specifically, the temperature just below the catalyst bed was measured to assume that the temperature before the feedstocks flowed was the temperature of the vaporization chamber/reaction tube and that the temperature during the flow of the feedstocks was the reaction temperature (contact temperature).

### [Detection of unknown component]

A case in which a compound (unknown component) for which the molecular structure cannot be determined was detected by the measurement method for the product of the contact reaction (dehydration reaction) described in the above section [Analytical conditions] is designated as "Present", while a case in which no unknown component was detected is designated as "Absent".

**[Table 1]**

| | Example 1 | Example 2 | Example 3 | Example 4 | Example 5 | Example 6 | Example 7 | Example 8 | Example 9 | Example 10 | Comparative example 1 | Comparative example 2 | Comparative example 3 | Comparative example 4 | Comparative example 5 | Comparative example 6 | Comparative example⁷ |
|---|---|---|---|---|---|---|---|---|---|---|---|---|---|---|---|---|---|
| Diluent type | 1.3,5-TMB | 1.3.5-TMB | 1.3.5-TMB | 1.3.5-TMB | 1.3,5-TMB | 1.3,5-TMB | 1.3.5-TMB | 1.3.5-TMB | 1.3,5-TlIB | 1.3.5-TMB | 1.3.5-TMB | 1.3.5-TMB | 1.3.5-TMB | 1.3.5-TMB | 1,3.5-TMB | 1.3.5-TMB | 1.3.5-TMB |
| Autoignition temperature of diluent °C | 559 | 559 | 559 | 559 | 559 | 559 | 559 | 559 | 559 | 559 | 559 | 559 | 559 | 559 | 559 | 559 | 559 |
| Reactor | Figure 2 | Figure 2 | Figure 2 | Figure 2 | Figure 2 | Figure 2 | Figure 2 | Figure 2 | Figure 2 | Figure 2 | Figure 2 | Figure 2 | Figure 2 | Figure 2 | Figure 2 | Figure 2 | Figure 2 |
| Inner diameter of reactor mm | 10.8 | 10.8 | 10.8 | 10.8 | 10.8 | 10.8 | 10.8 | 10.8 | 10.8 | 10.8 | 10.8 | 10.8 | 10.8 | 10.8 | 10.8 | 10.8 | 10.8 |
| Cross-sectional area of reactor mm² | 91.6 | 91.6 | 91.6 | 91.6 | 91.6 | 91.6 | 91.6 | 91.6 | 91.6 | 91.6 | 91.6 | 91.6 | 91.6 | 91.6 | 91.6 | 91.6 | 91.6 |
| Catalyst | Cs₂O SiO₂ (G-6) | Cs₂O SiO₂ (G-6) | Cs₂O/ SiO₂ (G-6) | Cs₂O/ SiO₂ (G-6) | Cs₂O SiO₂ (G-6) | Cs₂O/ SiO₂ (G-6) | Cs₂O/ SiO₂ (G-6) | Cs₂O/ SiO₂ (G-6) | Cs₂O/ SiO₂ (G-6) | Cs₂O SiO₂ (G-6) | Cs₂O SiO₂, (G-6) | Cs₂O SiO₂, (G-6) | Cs₂O/ SiO₂ (G-7) | Cs₂O /SiO₂ (G-8) | Cs₂O SiO₂ (G-9) | Cs₂O SiO₂ (G-10) | Cs₂O/ SiO₂ (G-6) |
| Amount of catalyst (Cs₂O/SiO₂) g | 1 | 1 | 1 | 1 | 1 | 1 | 1 | 1 | 1 | 0.62 | 1 | 1 | 1 | 1 | 1.07 | 1.1 | 1.1 |
| Temperature of vaporizer/reactor °C | 337 | 318 | 350 | 377 | 444 | 508 | 505 | 494 | 487 | 500 | 285 | 283 | 295 | 294 | 293 | 294 | 294 |
| Reaction temperature (catalyst bed) °C | 323.5 | 304.8 | 324.2 | 345.7 | 403.8 | 448.1 | 451.5 | 490.4 | 482.8 | 440.8 | 281.6 | 282.8 | 290.9 | 286 | 284 | 288.8 | 283.1 |
| Reaction pressure kPa | 41.5 | 41.6 | 40.7 | 40.1 | 39.8 | 39.6 | 38.9 | 20.5 | 40.2 | 101.3 | 40.4 | 40.8 | 40.9 | 40.8 | 40.3 | 39.5 | 41.7 |
| Flow rate of diluent mol min | 0.013 | 0.014 | 0.014 | 0.013 | 0.012 | 0 | 0.005 | 0.0047 | 0.0121 | 0.0179 | 0.013 | 0.006 | 0.002 | 0.003 | 0.012 | 0.009 | 0.023 |
| Flow rate of 2-PA mol min | 0.013 | 0.014 | 0.014 | 0.013 | 0.012 | 0.0234 | 0.02 | 0.0047 | 0.0121 | 0.0179 | 0.013 | 0.023 | 0.007 | 0.014 | 0.019 | 0.009 | 0.023 |
| Diluent / 2PA (mole ratio) | 1 | 1 | 1 | 1 | 1 | 0 | 0.27 | 1 | 1 | 1 | 1 | 0.25 | 0.25 | 0.25 | 0.67 | 1 | 1 |
| Feedstock 2-PA mol% % | 50 | 50 | 50 | 50 | 50 | 100 | 78.8 | 50 | 50 | 50 | 50 | 80 | 80 | 80 | 60 | 50 | 50 |
| Contact time sec | 0.181 | 0.172 | 0.159 | 0.159 | 0.156 | 0.155 | 0.139 | 0.19 | 0.145 | 0.157 | 0.18 | 0.167 | 0.545 | 0.279 | 0.154 | 0.266 | 0.11 |
| 2-PA conversion rate % | 35.9 | 24.5 | 31.1 | 42 | 70.7 | 64.3 | 67.7 | 72.6 | 97.3 | 64.7 | 19.5 | 13.6 | 26.4 | 17.5 | 13.7 | 22.9 | 12.3 |
| Percent yield of 2-CP % | 35.2 | 24.1 | 30.5 | 41.2 | 69.2 | 62.7 | 66.2 | 68.3 | 95.8 | 63.4 | 19.1 | 13.2 | 25.3 | 16.9 | 13.4 | 22.2 | 12.1 |
| Yield of 2-CP mol day | 6.44 | 4.78 | 6.22 | 8 | 12.41 | 21.15 | 19.21 | 4.6 | 16.68 | 16.35 | 3.67 | 4.41 | 2.56 | 3.37 | 3.58 | 2.79 | 3.95 |
| Percent yield of Py (%) % | 0.77 | 0.477 | 0.609 | 0.834 | 1.456 | 1.654 | 1.549 | 4.337 | 1.444 | 1.316 | 0.371 | 0.35 | 1.129 | 0.545 | 0.373 | 0.737 | 0.197 |
| By-product rate of Py % | 2.19 | 1.98 | 1.99 | 2.03 | 2.1 | 2.64 | 2.34 | 6.35 | 1.51 | 2.08 | 1.94 | 2.65 | 4.46 | 3.21 | 2.8 | 3.32 | 1.62 |
| Presence or absence of UK detection | Absent | Absent | Absent | Absent | Absent | Absent | Absent | Absent | Absent | Absent | Absent | Absent | Absent | Absent | Absent | Absent | Absent |

### Example 11 (Example using solvent as diluent)

SiO₂ (from Fuji Silicia Chemical, CARiACT, G-6 (support diameter 0.010 mm)) as a support was pre-calcined at 700°C for 1 hour. Subsequently, to support Cs as an alkali metal, an aqueous solution was prepared using CsNO₃ (from Wako Pure Chemical Industries, Ltd.) so that the final amount of the supported Cs metal was 0.5 mmol/g, and the aqueous solution was used to impregnate SiO₂. The resultant was then dried at 110°C for 6 hours and calcined at 500°C for 3 hours to obtain a Cs₂O/SiO₂ catalyst.

The catalyst produced by the method described above was packed into a reaction tube 36 made of SUS316 with an inner diameter of 10.8 mm and a length of 35 cm (see Figure 2). Furthermore, a vaporization chamber 33 made of SUS316 with an inner diameter of 10.8 mm and a length of 35 cm, packed with Raschig rings, was placed directly above the reaction tube 36.

170 g of 2-picolinamide (2-PA) was heated and mixed with p-cymene (187 g) as a transfer solvent in an oil bath at 140°C. The resulting mixed solution of 2-PA and p-cymene was transferred to a feedstock vessel 30.

The vaporization chamber 33, connected to the feedstock vessel 30, and the reaction tube 36 were heated in tube furnaces 34 and 35 (see Figure 2). The outlet pipe of the reaction tube 36 was kept warm at 180°C. A dry ice/methanol cooling trap vessel 37 and a trap vessel 38 were installed to collect the reactants. The outlet pipe of the trap vessel 38 was connected to a vacuum pump 39, and the pressure in the vaporization chamber 33 and the reaction tube 36 was reduced to 40 (KPa).

After the temperature of the vaporization chamber and the reaction tube was stabilized at 382°C, a plunger pump 32 was initiated to carry out the reaction for the time indicated in the column "Catalyst contact time" in Table 1 below.

### Examples 12-13 and Comparative examples 8-15

Using the same reactor as in Example 11, a dehydration reaction of 2-picolinamide was carried out in the same manner as in Example 11, except that the reaction conditions were changed as shown in Table 2 below.

### Example 14

SiO₂ (from Fuji Silicia Chemical, CARiACT, G-6 (support diameter 0.010 mm)) as a support was pre-calcined at 700°C for 1 hour. Subsequently, to support K (potassium) as an alkali metal, an aqueous solution was prepared using KNO₃ (from Wako Pure Chemical Industries, Ltd.) so that the final amount of the supported K metal was 0.5 mmol/g, and the aqueous solution was used to impregnate SiO₂. The resultant was then dried at 110°C for 6 hours and calcined at 500°C for 3 hours to obtain a K₂O/SiO₂ catalyst.

The catalyst produced by the method described above was packed into a reaction tube 36 made of SUS316 with an inner diameter of 10.8 mm and a length of 35 cm (see Figure 2). Furthermore, a vaporization chamber 33 made of SUS316 with an inner diameter of 10.8 mm and a length of 35 cm, packed with Raschig rings, was placed directly above the reaction tube 6.

203 g of 2-picolinamide (2-PA) was heated and mixed with 1,3,5-trimethylbenzene (200 g) as a transfer solvent in an oil bath at 140°C. The resulting mixed solution of 2-PA and 1,3,5-trimethylbenzene was transferred to a feedstock vessel 30.

The vaporization chamber 33, connected to the feedstock vessel 30, and the reaction tube 36 were heated to 511.2°C in tube furnaces 34 and 35 (see Figure 2). The outlet pipe of the reaction tube 36 was kept warm at 180°C. A dry ice/methanol cooling trap vessel 37 and a trap vessel 38 were installed to collect the reactants. The outlet pipe of the trap vessel 38 was connected to a vacuum pump 39, and the pressure in the vaporization chamber 33 and the reaction tube 36 was reduced to 40 (KPa).

After the temperature of the catalyst bed was stabilized, a plunger pump 32 was initiated to carry out the reaction for the time indicated in the column "Catalyst contact time" in Table 2 below.

### Example 15

SiO₂ (from Fuji Silicia Chemical, CARiACT, G-6 (support diameter 0.010 mm)) as a support was pre-calcined at 700°C for 1 hour. Subsequently, to support Na as an alkali metal, an aqueous solution was prepared using NaNO₃ (from Wako Pure Chemical Industries, Ltd.) so that the final amount of the supported Na metal was 0.5 mmol/g, and the aqueous solution was used to impregnate SiO₂. The resultant was then dried at 110°C for 6 hours and calcined at 500°C for 3 hours to obtain a Na₂O/SiO₂ catalyst.

The catalyst produced by the method described above was packed into a reaction tube 36 made of SUS316 with an inner diameter of 10.8 mm and a length of 35 cm (see Figure 2). Furthermore, a vaporization chamber 33 made of SUS316 with an inner diameter of 10.8 mm and a length of 35 cm, packed with Raschig rings, was placed directly above the reaction tube 36.

Using the same reactor as in Example 14, a dehydration reaction of 2-picolinamide was carried out in the same manner as in Example 14, except that the reaction conditions other than the catalyst were changed as shown in Table 2 below.

### Example 16 (Example using inert gas (nitrogen gas) as diluent)

SiO₂ (from Fuji Silicia Chemical, CARiACT, Q-6 (support diameter 0.075-0.15 mm)) as a support was pre-calcined at 700°C for 1 hour. Subsequently, to support Cs as an alkali metal, an aqueous solution was prepared using CsNO₃ (from Wako Pure Chemical Industries, Ltd.) so that the final amount of the supported Cs metal was 0.5 mmol/g, and the aqueous solution was used to impregnate SiO₂. The resultant was then dried at 110°C for 6 hours and calcined at 500°C for 3 hours to obtain a Cs₂O/SiO₂ catalyst.

The catalyst produced by the method described above was packed into a reaction tube 36 made of SUS316 with an inner diameter of 10.8 mm and a length of 35 cm (see Figure 3). Furthermore, a vaporization chamber 33 made of SUS316 with an inner diameter of 10.8 mm and a length of 35 cm, packed with Raschig rings, was placed directly above the reaction tube 36.

100 g of 2-picolinamide (2-PA) was dissolved in an oil bath at 140°C, and 2-PA was transferred to the feedstock vessel 30 without the use of a solvent.

The vaporization chamber 33, connected to the feedstock vessel 30, and the reaction tube 36 were heated in tube furnaces 34 and 35 (see Figure 3). The outlet pipe of the reaction tube 36 was kept warm at 180°C. A dry ice/methanol cooling trap vessel 37 and a trap vessel 38 were installed to collect the reactants. The outlet side of the trap vessel 38 was open to the atmosphere.

After the temperature of the vaporization chamber and the reaction tube was stabilized at 516°C, a plunger pump 32 was initiated and nitrogen gas was simultaneously flowed in as a diluent to carry out a reaction for the time indicated in the column "Catalyst contact time" in Table 2 below.

### Comparative example 16

Using the same reactor as in Example 16 above, a dehydration reaction of 2-picolinamide was carried out in the same manner as in Example 16, except that the reaction conditions were changed as shown in Table 2 below.

**[Table 2]**

| | Example 11 | Example 12 | Example 13 | Example 14 | Example 15 | Example 16 | Comparative example 8 | Comparative example 9 | Comparative example 10 | Comparative example 11 | Comparative example 12 | Comparative example 13 | Comparative 14 | Comparative example 15 | Comparative example 16 |
|---|---|---|---|---|---|---|---|---|---|---|---|---|---|---|---|
| Diluent type | | Phenyl ether | Anisole | 1.3,5-TMB | 1.3,5-TMB | N₂ | - | 1.3,5-TMB | Phenyl ether | Anisole | 2-Octanone | 3.3.5- | example Acetophenone | Cineole | N₂ |
| Autoignition temperature of diluent °C | 436 | 617 | 475 | 559 | 559 | - | 436 | 559 | 617 | 475 | 380 | 473 | 571 | 300 | - |
| Reactor | Figure 2 | Figure 2 | Figure 2 | Figure 2 | Figure 2 | Figure 3 | Figure 2 | Figure 2 | Figure 2 | Figure 2 | Figure 2 | Figure 2 | Figure 2 | Figure 2 | Figure 3 |
| Inner diameter of reactor mm | 10.8 | 10.8 | 10.8 | 10.8 | 10.8 | 10.8 | 9.4 | 9.4 | 9.4 | 9.4 | 9.4 | 9.4 | 9.4 | 9.4 | 10.8 |
| Cross-sectional area of reactor mm² | 91.6 | 91.6 | 91.6 | 91.6 | 91.6 | 91.6 | 69.4 | 69.4 | 69.4 | 69.4 | 69.4 | 69.4 | 69.4 | 69.4 | 91.6 |
| Catalyst | Cs₂O SiO₂ (G-6) | Cs₂O SiO₂ (G-6) | Cs₂O SiO₂ (G-6) | K₂O SiO₂ (G-6) | Na₂O SiO₂ (G-6) | Cs₂O SiO₂ (Q-6) | Cs₂O SiO₂ (G-6) | Cs₂O SiO₂ (G-6) | Cs₂O SiO₂ (G-6) | Cs₂O SiO₂ (G-6) | CS₂O SiO₂ *(G-6)* | Cs₂O SiO₂ (G-6) | Cs₂O SiO₂ (G-6) | Cs₂O SiO₂ *(G-6)* | Cs₂O SiO₂ (Q-6) |
| Amount of catalyst g | 0.98 | 0.98 | 0.98 | 1 | 1 | 9 | 1 | 1 | 1 | 1 | 1 | 1 | 1 | 1 | 9 |
| Temperature of vaporizer/reactor °C | 382 | 508 | 403 | 511 | 513 | 516 | 280 | 282 | 281 | 281 | 278 | 281 | 280 | 280 | 248 |
| Reaction temperature (catalyst bed) °C | 341.8 | 473.9 | 362.9 | 474.3 | 476.2 | 481.2 | 278.8 | 276.2 | 277.1 | 275.3 | 277.3 | 278.3 | 276.9 | 278.6 | 247 |
| Reaction pressure kPa | 39.7 | 39.3 | 39.4 | 39.4 | 40 | 101.3 | 46.8 | 46.8 | 45.9 | 48.9 | 46.8 | 47.7 | 46.8 | 46.8 | 101.3 |
| Flow rate of diluent mol min | 0.014 | 0.012 | 0.015 | 0.0119 | 0.0119 | 0.0446 | 0.007 | 0.008 | 0.007 | 0.009 | 0.007 | 0.007 | 0.008 | 0.007 | 0.0446 |
| Flow rate of 2-PA mol min | 0.014 | 0.012 | 0.015 | 0.0119 | 0.0119 | 0.0015 | 0.007 | 0.008 | 0.006 | 0.009 | 0.007 | 0.007 | 0.008 | 0.007 | 0.0015 |
| Diluent 2PA (mole ratio) | 1 | 1 | 1 | 1 | 1 | 29.73 | 1 | 1 | 1 | 1 | 1 | 1 | 1 | 1 | 29.73 |
| Feedstock 2-PA mol% % | 50 | 50 | 50 | 50 | 50 | 3.3 | 50 | 50 | 50 | 50 | 50 | 50 | 50 | 50 | 3.3 |
| Contact time sec | 0.165 | 0.145 | 0.137 | 0.15 | 0.15 | 0.813 | 0.074 | 0.069 | 0.078 | 0.063 | 0.078 | 0.077 | 0.069 | 0.076 | 0.813 |
| 2-PA conversion rate % | 49.8 | 95.4 | 61.5 | 94.2 | 93.8 | 88.3 | 28.3 | 26.3 | 28.3 | 24.9 | 26.1 | 20.2 | 24.7 | 27.7 | 25.6 |
| Percent yield of 2-CP % | 48.98 | 93.7 | 60.5 | 91.6 | 88.5 | 88.3 | 27.3 | 25.4 | 27.2 | 24.1 | 25.3 | 19.7 | 24 | 26.9 | 25.6 |
| Yield of 2-CP mol day | 9.13 | 16.2 | 13.01 | 15.2 | 14.7 | 2.1 | 2.73 | 2.74 | 2.54 | 2.98 | 2.42 | 1.95 | 2.59 | 2.63 | 0.6 |
| Percent yield of Py (%) % | 0.833 | 1.648 | 0.984 | 2.68 | 4.4 | 0 | 0.993 | 0.943 | 1.128 | 0.814 | 0.81 | 0.502 | 0.693 | 0.812 | 0 |
| By-product rate of Py % | 1.7 | 1.76 | 1.63 | 2.93 | 4.96 | 0 | 3.64 | 3.72 | 4.15 | 3.38 | 3.2 | 2.55 | 2.89 | 3.02 | 0 |
| Presence or absence of UK detection | Absent | Absent | Absent | Absent | Absent | Absent | Absent | Absent | Absent | Absent | Present | Present | Present | Absent | Absent |

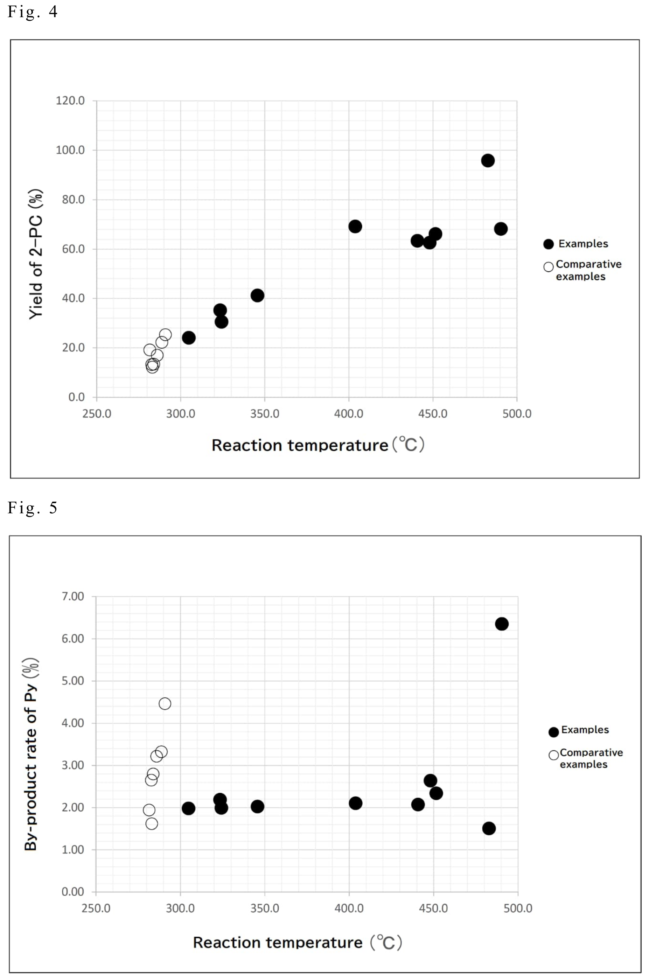

As can be appreciated from the results of the examples and comparative exmaples, the higher the reaction temperature in the gas-phase dehydration reaction, the higher the percent yield of the aromatic nitrile compound. There was also a tendency to suppress the by-product rate of the by-product pyridine (see Tables 1 and 2 above and Figures 4 and 5). Although a relatively large amount of by-products were generated in some of the examples, the overall evaluation was good for all of the examples due to the high percent yield of the aromatic nitrile compound.

Although preferable embodiments of the present invention have been described in detail with reference to the accompanying drawings, the present invention is not limited to the above examples. A person having ordinary skill in the art to which the present invention pertains would obviously conceive of various alterations and modifications within the scope of the technical ideas defined by the claims, and such alterations and modifications should be construed as being duly within the technical scope of the present invention.

### DESCRIPTION OF REFERENCE NUMERALS

30 Feedstock vessel
32 Plunger pump
33 Vaporization chamber
34 Double pipe heat exchanger/tube furnace
35 Double pipe heat exchanger/tube furnace/mantle heater
36 Reaction tube
37 Cooling trap vessel
38 Trap vessel
39 Vacuum pump
40 Nitrogen introduction line

## Claims

1. A method for producing an aromatic nitrile compound, comprising a dehydration reaction for dehydrating an aromatic amide compound, wherein
the dehydration reaction comprises a contact step in which the aromatic amide compound and a diluent are brought into contact with a catalyst in a gas phase, and
the temperature at which the aromatic amide compound and the diluent are brought into contact with the catalyst in the contact step is 300°C or higher.

2. The method for producing an aromatic nitrile compound according to claim 1, wherein the diluent comprises at least either a solvent or an inert gas.

3. The method for producing an aromatic nitrile compound according to claim 2, wherein the solvent comprises at least an aromatic compound.

4. The method for producing an aromatic nitrile compound according to claim 3, wherein the aromatic compound is represented by the following formula (1): 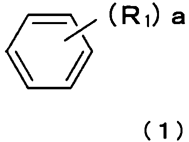 in formula (1),
R₁ is each independently selected from an optionally substituted C1-C10 alkyl group, an optionally substituted C1-C10 alkoxy group, an optionally substituted C6-C30 aryl group, and an optionally substituted C6-C30 aryloxy group, and
a is an integer from 1 to 6.

5. The method for producing an aromatic nitrile compound according to claim 3, wherein the aromatic compound has one or two aromatic rings and one or more selected from an alkyl group, an alkoxy group, and an aryloxy group.

6. The method for producing an aromatic nitrile compound according to claim 3, wherein the aromatic compound has an autoignition temperature of 300°C or higher.

7. The method for producing an aromatic nitrile compound according to claim 3, wherein the aromatic compound comprises at least one of 1,3,5-trimethylbenzene, p-cymene, phenyl ether, and anisole.

8. The method for producing an aromatic nitrile compound according to claim 1, wherein the pressure of a reaction system in the contact step is 101.3 kPa or less.

9. The method for producing an aromatic nitrile compound according to claim 1, wherein the aromatic amide compound comprises at least a heteroaryl amide compound, and the aromatic nitrile compound comprises at least a heteroaryl nitrile compound.

10. The method for producing an aromatic nitrile compound according to claim 9, wherein the heteroaryl amide compound comprises 2-picolinamide, and the heteroaryl nitrile compound comprises 2-cyanopyridine.

11. The method for producing an aromatic nitrile compound according to claim 1, wherein an inert gas in a vaporized state is used in the contact step.

12. The method for producing an aromatic nitrile compound according to claim 11, wherein the inert gas comprises at least nitrogen gas.

13. The method for producing an aromatic nitrile compound according to claim 1, wherein the catalyst comprises an alkali metal.

14. The method for producing an aromatic nitrile compound according to claim 1, wherein the solvent is compatible with the aromatic amide compound.

15. The method for producing an aromatic nitrile compound according to claim 1, wherein the contact time of the aromatic amide compound with the catalyst in the gas phase is 0.001 seconds or more but less than 10 seconds.

16. A method for producing a carbonate ester, comprising:
a first reaction step comprising a carbonate ester-generating reaction in which an alcohol and carbon dioxide are reacted with each other in the presence of an aromatic nitrile compound to generate a carbonate ester and water, and a hydration reaction in which the aromatic nitrile compound is hydrated with the generated water to form an aromatic amide compound; and
a second reaction step in which the aromatic amide compound is separated from the reaction system of the first reaction step, and then an aromatic nitrile compound is regenerated from the aromatic amide compound by a dehydration reaction for dehydrating the aromatic amide compound, the dehydration reaction involving the contact step according to any one of claims 1-15,
wherein at least a portion of the aromatic nitrile compound regenerated in the second reaction step is used in the first reaction step.

17. The method for producing a carbonate ester according to claim 16, wherein a catalyst comprising cerium oxide is used in the carbonate ester-generating reaction.

18. The method for producing a carbonate ester according to claim 16, wherein the alcohol comprises a C1-C6 alcohol.
